# EUROPÄISCHE PATENTSCHRIFT

(11) **EP 0 829 712 B1**
(45) Veröffentlichungstag und Bekanntmachung des Hinweises auf die Patenterteilung: **26.05.2004**
(21) Anmeldenummer: 97115676.5
(22) Anmeldetag: 10.09.1997
(51) Int. Cl.: G01N 33/00, G01N 25/18, C12M 1/36, C12M 1/00

(54) **Verfahren zum Nullpunktabgleich einer Wärmeleitfähigkeits-Messzelle zur CO2-Messung in einem Begasungsbrutschrank**
Method for offsetting a heat conductivity measuring cell for measuring CO2 in an incubator with gas circulation
Méthode de décalage du zéro d'une cellule de mesure de la conductivité de la chaleur pour la mesure du CO2 dans un incubateur à circulation de gaz

(30) Priorität: 13.09.1996 DE 19637520
(43) Veröffentlichungstag der Anmeldung: 18.03.1998
(73) Patentinhaber: KENDRO Laboratory Products GmbH, 63450 Hanau (DE)
(72) Erfinder: Hessler, Karl-Heinz, 63776 Mömbris (DE); Heeg, Hubert, 63776 Mömbris (DE)
(74) Vertreter: Lang, Friedrich

(56) Entgegenhaltungen:
- DE-A- 3 315 085
- US-A- 3 848 569
- US-A- 3 929 584
- US-A- 5 418 131

## Beschreibung

Die Erfindung betrifft ein Verfahren zum Nullpunktabgleich einer Wärmeleitfähigkeits-Meßzelle in einem Begasungsbrutschrank mit einer CO₂ enthaltenden Nutzraumatmosphäre, die mit Hilfe der Meßzelle geregelt wird, wie im Oberbegriff des Anspruchs 1 beschrieben ist.

Die US-A-3,929,584 beschreibt den Aufbau einer Wärmeleitfähigkeitsmesszelle in einem Inkubator (Begasungsbrutschrank). Die Wärmeleitfähigkeitsmesszelle besteht als Differentialthermometer aus zwei Heißleitersensoren, einem Referenzheißleiter und einem Meßheißleiter, die über eine Wheatstone'sche Brücke zusammengeschaltet sind. Der Referenzheißleiter bzw. der Meßheißleiter wird über getrennte Leitungen mit jeweils einer eigenen Vakuumpumpe mit Referenzgas bzw. Heizgas kontinuierlich beaufschlagt. Aufgrund der gemessenen Differenz zwischen den Heißleitern kann die CO₂-Begasung automatisch und kontinuierlich um einen einstellbaren Wert zur Erhaltung einer vorher festgelegten CO₂-Konzentration geregelt werden.

Aus der DE 33 15 085 C2 ist ein Verfahren zur Nullpunktkontrolle an Wärmeleitfähigkeitsmeßzellen in Begasungsbrutschränken mit einer CO₂-enthaltenden Nutzraumatmosphäre bekannt, die mit Hilfe der Wärmeleitfähigkeitsmeßzellen regelbar sind, wobei der Meßseite des Wärmeleitfähigkeitsdetektors stetig Nutzraumgasatmosphäre zugeführt wird. Ein Begasungsbrutschrank solcher Art ist beispielsweise aus der DE 29 24 446 C2 bekannt.

Die Wärmeleitfähigkeitsmeßzellen weisen eine Referenzseite auf, wobei während einer Meßphase Luft oder ein anderes externes Gas als Referenzgas zugeführt wird; während einer Eichphase wird diese Zufuhr unterbrochen und die Referenzseite der Nutzraumatmosphäre ausgesetzt; das sich dann einstellende Signal wird gespeichert und in der Meßphase vom über die Meßseite der Wärmeleitfähigkeitsmeßzelle erhaltenen Meßsignal subtrahiert, um so ein Nullpunkt-Korrektursignal zu erhalten bis zur jeweiligen darauf folgenden nächsten Eichphase, wobei dann dieses Korrektursignal durch ein neues Nullpunkt-Korrektursignal ersetzt wird.

Als problematisch erweist sich der verhältnismäßig aufwendige Aufbau eines solchen Begasungsbrutschrankes, da die Referenzseite sowohl mit der Nutzraumatmosphäre als auch mit einem Referenzgas - Volumen - wie z. B. der Außenatmosphäre zu verbinden ist, wobei während des Betriebes auf jeden Fall Undichtigkeiten vermieden werden müssen, damit nicht sowohl die Meß- als auch die Referenzseite der gleichen Gasatmosphäre ausgesetzt sind; weiterhin stellt der Abgleich keinen absoluten Nullpunkt-Abgleich der gesamten Meßzelle dar, sondem bildet lediglich ein Signal zur Korrektur der Drift der Sensoren.

Ein ähnlicher Begasungsbrutschrank ist beispielsweise in US-A-5,418,131 offenbart. Die Patentschrift'131 betrifft ein feuchtigkeitskompensiertes CO₂-Meß- und Regelungssystem mit einem CO₂-Wärmeleitfähigkeitssensor, einem Feuchtigkeitssensor und einem Temperatursensor. Diese Sensoren erzeugen unabhängige Ausgabesignale, die von einem Mikroprozessor zu einem feuchtigkeitskompensierten und -korrigierten CO₂-Meßwert verarbeitet werden. Hierzu sind in einer Referenztabelle spezifischen Feuchtigkeitswerten kalibrierte CO₂-Nullpunktwerte zugewiesen. Der Mikroprozessor entnimmt aus dieser abgespeicherten Referenztabelle einen CO₂-Nullpunktwert entsprechend der aufgenommenen Ausgabesignale des Feuchtigkeits- und Temperatursensors und gleicht dann den gegenwärtigen CO₂-Meßwert um den aus der Referenztabelle entnommenen CO₂-Nullpunktwert ab. Die Differenz zwischen feuchtigkeitskompensiertem CO₂-Meßwert und CO₂-Nullpunktwert dient zur Regelung der CO₂-Begasung um einen festgelegten CO₂-Konzentrationswert.

Aus der EP 154 536 A2 ist ein Inkubator für Laboranwendungen bekannt, der ein Gehäuse mit thermisch isolierter Innenkammer aufweist, die eine kontrollierte Gasatmosphäre enthält; die Gasatmosphäre der Inkubations-Kammer zirkuliert durch einen außerhalb der Kammer befindlichen Rezirkulationspfad, der ein Gebläse, ein Filter, einen CO₂-Sensor und eine Befeuchtungsanlage aufweist; einem mikroprozessorgesteuerten Regler werden Signale von der CO₂-Messung, der Temperatur-Messung, sowie der Feuchte-Messung zugeführt, wobei der Regler seinerseits wiederum Stellsignale für Gebläse, CO₂-Ventil, Wasserzufuhr zur Befeuchtungsanlage, Aufheizeinrichtung abigbt. Innerhalb der Inkubations-Kammer kann somit auf kontaminierbare Sensoren und Umwälzeinrichtungen verzichtet werden, wobei innerhalb der Kammer eine laminare Strömung vorherrscht.

Eine besondere Justierung des CO₂-Sensors ist nicht vorgesehen.

Aufgabe der Erfindung ist es, ein Verfahren zur automatischen Nullpunktkontrolle anzugeben, das sich auch für jeden Sensoraufbau eignet, bei dem nur im Inneren des Nutzraumes Messungen stattfinden, wobei die Nullpunktjustage auch im Anschluß an einen Desinfektionsvorgang bei 180°C einsetzbar ist.

Weiterhin soll ein möglichst stabiler Langzeitbetrieb im Nutzraum möglich sein.

Die Aufgabe wird dadurch mit den Merkmalen des Anspruchs 1 gelöst.

Als besonders vorteilhaft erweist sich die einfache Bedienung des Gerätes, da Inbetriebnahme und Abgleich auch automatisch - beispielsweise nach einer Desinfektion - erfolgen kann; nach Ablauf dieser Inbetriebnahmeroutine kann das Gerät mit Laborproben direkt beschickt werden.

In einer vorteilhaften Ausgestaltung des Verfahrens werden nach Aufruf des Start-Schritts S1) alle Offset-Werte intern auf Null zurückgesetzt; dabei werden die ermittelten CO₂-Zellen-Offset-Werte vorzeichenrichtung stets zum CO₂-Meßwert hinzuaddiert.

Weitere vorteilhafte Ausgestaltungen des Verfahrens sind in den abhängigen Ansprüchen angegeben.

Dabei erweist sich die Vorgabe eines CO₂-Toleranzbereiches eines CO₂-Meßwertes im Bereich von ± 0,2 Vol-% CO₂ als für die Praxis gut geeignet.

Nachfolgend ist der Gegenstand der Erfindung anhand der Figuren 1, 2, 3a und 3b näher erläutert.
Figur 1 zeigt den Verfahrensablauf anhand eines Flußdiagramms,
Figur 2 zeigt den CO₂-Anzeigewert in Abhängigkeit des Zeitablaufs,
Figur 3a zeigt schematisch teilweise aufgebrochen im Schnitt einen Brutschrank mit eingebauter Wärmeleitfähigkeitsmeßzelle.
Figur 3b zeigt schematisch im Schnitt eine Wärmeleitfähigkeits-Meßzelle.

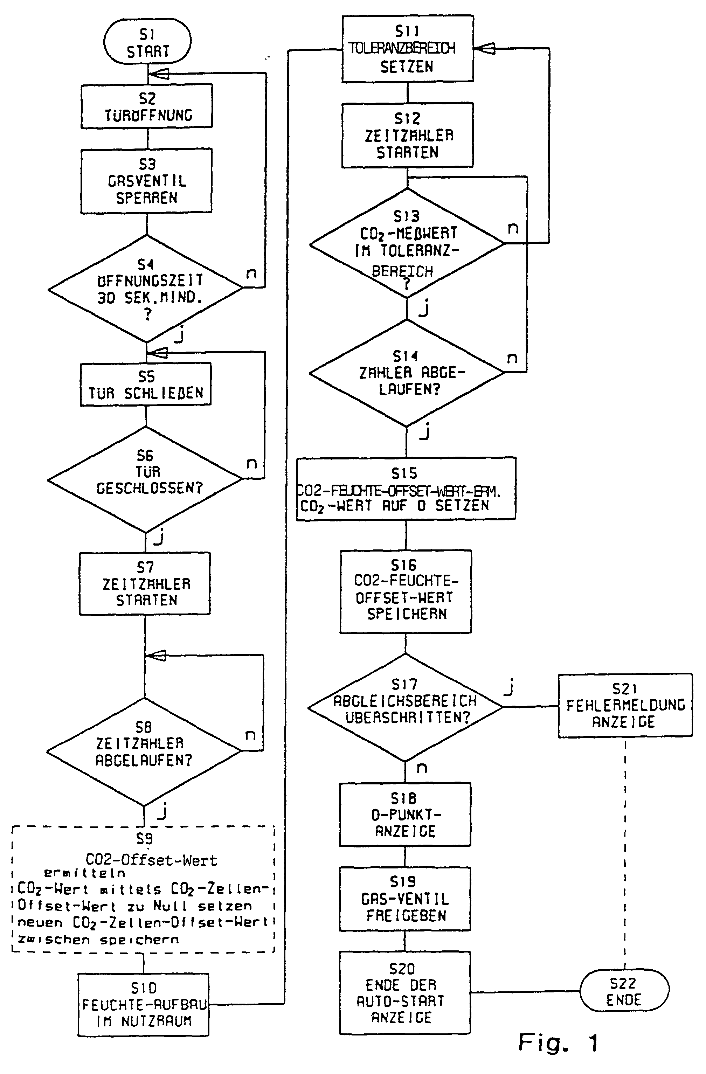

Gemäß Figur 1 beginnt in Schritt S1) der Start des Verfahrens zum Nullpunktabgleich an einer Wärmeleitfähigkeitsmeßzelle; es ist möglich, den Schritt S1) durch manuelle Betätigung auszulösen, es ist jedoch auch möglich, den Schritt S1) nach Ablauf eines Desinfektionsvorganges des Begasungsbrutschrankes oder nach Durchführung eines fehlerhaften Nullpunktabgleichdurchganges automatisch durchzuführen.

Auf Schritt S1) folgt Schritt S2), die Anzeige "Türöffnung", um einen Zutritt der atmosphärischen Umgebungsluft als Referenzgas in den Innenraum des Brutschrankes zu ermöglichen; parallel erfolgt Schritt S3) "Gasventile sperren", diese Zeit sollte mindestens 30 Sekunden betragen; im Abfrage-Schritt S4) wird die Öffnungszeit von 30 Sekunden überprüft und bei Nichterfüllung erneut die Anzeige zur Türöffnung ausgegeben.

Bei Erfüllung der Mindestöffnungszeit wird anschließend die Anzeige "Tür schließen" im Schritt S5) ausgegeben. Bei Nichterfüllung der Abfrage nach geschlossener Tür gemäß Schritt S6) erscheint erneut die Anzeige nach dem Schritt S5); bei Erfüllung der Abfrage S6) wird in Schritt S7) der Zeitzähler für die Ermittlung des CO₂-Offset-Wertes gestartet. In der Abfrage S8) wird überprüft, ob der Zeitzähler nach einer vorgegebenen Zeit abgelaufen ist und bei Erfüllung wird im Schritt S9) der CO₂-Offset-Wert ermittelt, der CO₂-Wert mittels CO₂-Zellen-Offset-Wert zu Null gesetzt und der neue CO₂-Zellen-Offset-Wert gespeichert. Im sich anschließenden Schritt S10) wird der Nutzraum bis zum Erreichen eines vorgegebenen Feuchtesollwertes im Bereich von 60 bis 95 % rF bedampft und nach Feuchteaufbau ein Toleranzband im Schritt S11) gesetzt, welches für die nachfolgende Ermittlung des CO₂-Feuchte-Offset-Werts dient. Im Schritt S12) wird ein Zeitzähler mit vorgegebener Zeit gestartet, wobei in Abfrage S13) geprüft wird, ob der CO₂-Meßwert im Toleranzbereich liegt oder nicht. Bei Nichterfüllung geht der Programmablauf zum Schritt S11) "Toleranzbereich setzen" zurück, bei Erfüllung wird im Abfrage-Schritt S14) geprüft, ob der Zähler abgelaufen ist, und falls dies zutrifft wird im Schritt S15) der CO₂-Wert auf Null gesetzt, wobei gleichzeitig im Schritt S16) der ermittelte CO₂-Feuchte-Offset-Wert gespeichert wird. Es sind somit sowohl der CO₂-Zellen-Offset-Wert als auch der CO₂-Feuchte-Offset-Wert gespeichert; falls beim Abfrage Schritt S14 der Zähler noch nicht abgelaufen ist, wird erneut im Schritt S13 gefragt, ob der CO₂-Meßwert im Toleranzbereich liegt.

In der Abfrage des Schritts S17) wird geprüft, ob der Abgleichsbereich überschritten ist, falls dies nicht zutrifft, erscheint im Schritt S18) die CO₂-Nullpunktanzeige und im Schritt S19) werden die Gasventile für die Begasung des Inneren des Brutschrankes freigegeben, im Schritt S20) erfolgt die Anzeige, daß der Startvorgang für den Nullpunktabgleich erfolgreich beendet ist. Das Programm geht zum Ende im Schritt S22) zurück.

Falls in der Abfrage S17) der Abgleichsbereich überschritten ist, erfolgt im Schritt S21) Fehlermeldungsanzeige, das Programm kehrt dann ggf. zum Schritt S1) mit einem neuen Start zurück, es ist jedoch auch möglich, das Programm mit Schritt S22) zu weiten (hinsichtlich des Abgleichsbereichs).

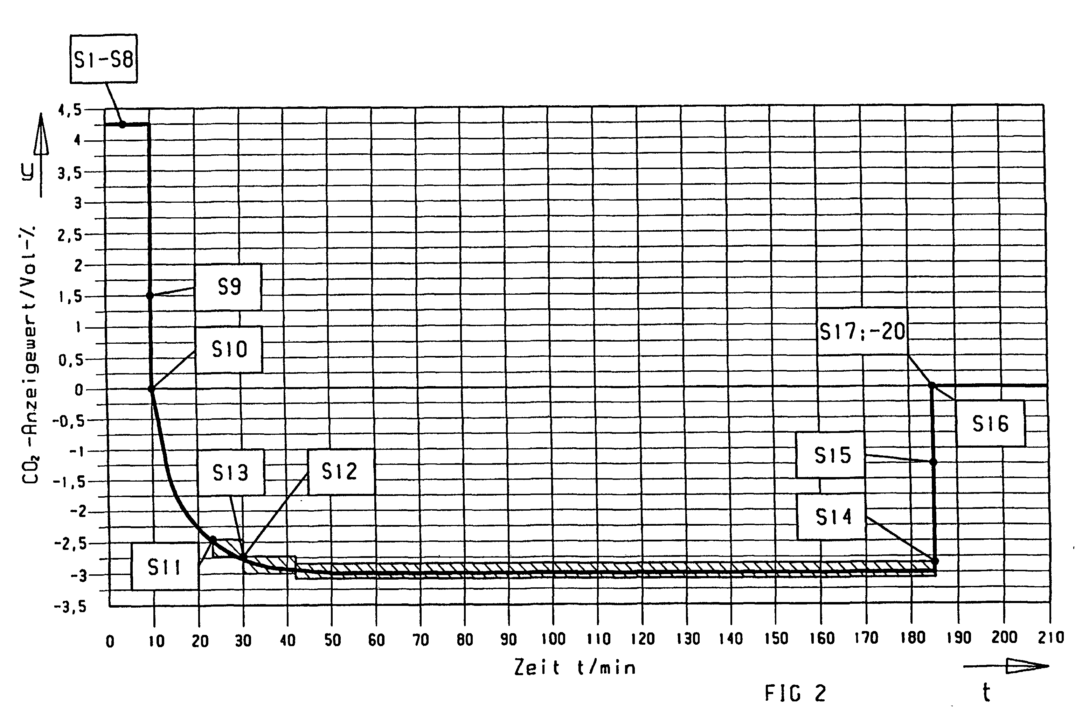

Anhand des Ablaufdiagramms nach Figur 2 wird beispielhaft der automatische Nullpunkt- Abgleich und die Ermittlung des Bezugs-Offsets einer CO₂-Wärmeleitfähigkeitsmeßzelle dargestellt.

Gemäß Figur 2 wird der CO₂-Anzeigewert y in Volumen-% über der Zeitachse t angegeben. Nachfolgend sind die Schritte gemäß Figur 1 mit beispielhaften Zeiten und beispielhaften Volumen-% näher erläutert; die nachfolgende Erläuterung bezieht sich somit auf Volumen-% für die Werte y und auf Minutenangaben für die Zeit t.

Nach Figur 2 werden im Zeitraum t = 0 bis 10 die Schritte S1), S2), S3), S4), S5), S6), S7) und S8) durchgeführt, bzw. abgefragt; falls der Zeitzähler im Schritt S8) abgelaufen ist, wird bei t = 10 der Schritt S9) vorgenommen, d.h. der Zellen-CO₂-Offset-Wert ermittelt; damit wird der CO₂-Wert auf Null gesetzt. Im Zeitpunkt t = 10 erfolgen Verdampfer-Check und Feuchteaufbau gemäß Schritt S10) im Inneren des Brutschrankes, wobei nunmehr aufgrund der durch die Feuchte erzeugten höheren Wärmeleitfähigkeit ein negativer CO₂-Anzeigewert im Bereich bis zu -3 Volumen-% erscheint, da Feuchtewert und CO₂-Anteil jeweils gegenläufig auf die als Sensor dienende Wärmeleitfähigkeitszelle einwirken; d.h. daß ein erhöhter CO₂-Gehalt aufgrund der Wärmeisolation von CO₂-Gas im Inneren des Brutschrankes eine verringerte Wärmeleitfähigkeit der Atmosphäre erzeugt, während die Wärmeleitfähigkeit bei Erhöhung des Feuchtegehaltes zunimmt und damit eine Abnahme des CO₂-Gehaltes vorgetäuscht wird. Anschließend nach Erreichen des Feuchtefehlerbandes erfolgt der Start des Toleranzbereiches mit einer Toleranz von +/- 0,2 Volumen-% CO₂, wobei dieser Toleranzbereich, welcher nach Figur 1 in Schritt S11) gesetzt worden ist, nach Abfrage S13) hierbei nicht eingehalten wird und der Toleranzbereich erneut gesetzt wird und auch Zeitzähler S12) neu gestartet werden muß. Auch nach dem Neustart des Toleranzbereiches im Bereich von t = 32 bis t = 42 wird das CO₂-Meßwerttoleranz- band gemäß Abfrage S13) nach Figur 1 nicht eingehalten, so erfolgt ein neuer Start im Zeitpunkt t = 42; d.h. Schritte S11, S12) nach Figur 1 werden wiederholt. Über die Gesamtdauer von t = 42 bis t = 185 des in Schritt S12) gestarteten Zeitzählers bleibt nunmehr der CO₂-Meß- wert im Toleranzbereich, so daß nach Ablauf des Zählers bei t = 185 gemäß Abfrage S14) der für die Ermittlung des CO₂-Feuchte-Offset-Wertes bedeutsame CO₂-Wert gemäß Schritt S15) auf Null gesetzt; im Schritt S16) dieser CO₂-Feuchte-Offset-Wert gespeichert wird.

In der Abfrage S17) wird geprüft, ob der Abgleichbereich überschritten worden ist; falls dies nicht zutrifft, erfolgt im Schritt S18) die Überschreibung der Werte des vorhergehenden Auto-Startes und die Nullpunktanzeige zum Zeitpunkt t = 185, anschließend wird das Ende des Nullpunktabgleichs ausgegeben (Schritt S20) und die Gasventile freigegeben (S19).

Es ist nunmehr nach Ablauf der Nullpunktkontrolle und der Begasung auf den eingestellten Sollwert möglich, den Innenraum des Brutschrankes mit Laborproben zu bestücken, die einer Innenraumatmosphäre mit geregelter CO₂-Zugabe ausgesetzt werden.

Falls gemäß Abfrage S17) der Abgleichsbereich überschritten worden ist, erfolgt nach Schritt S21) Fehlermeldungsanzeige und das Programm kehrt zum Zeitpunkt t = 0 zurück.

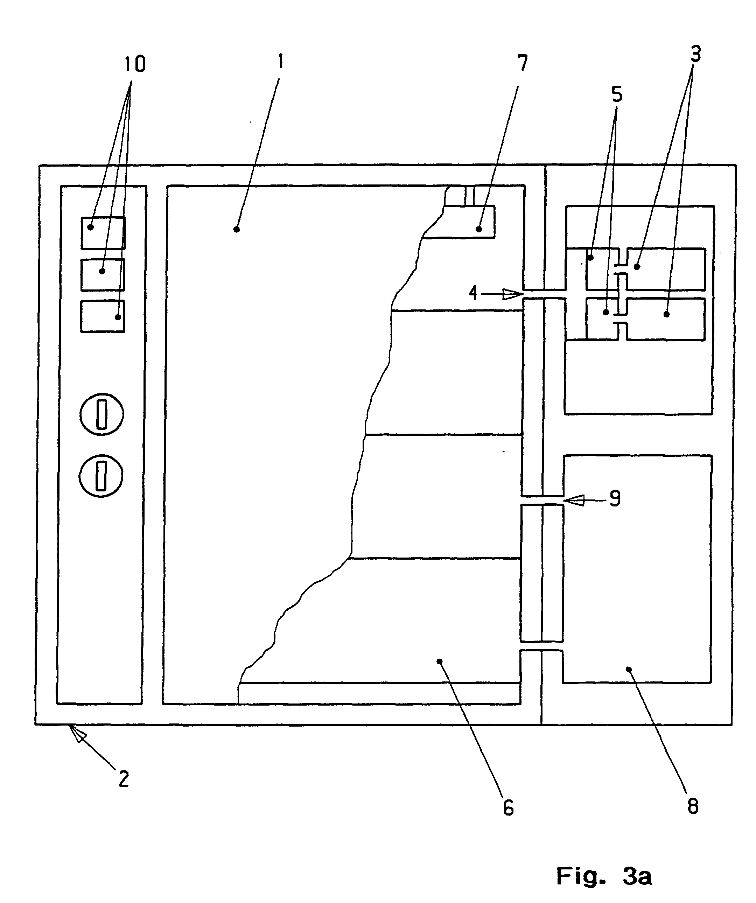

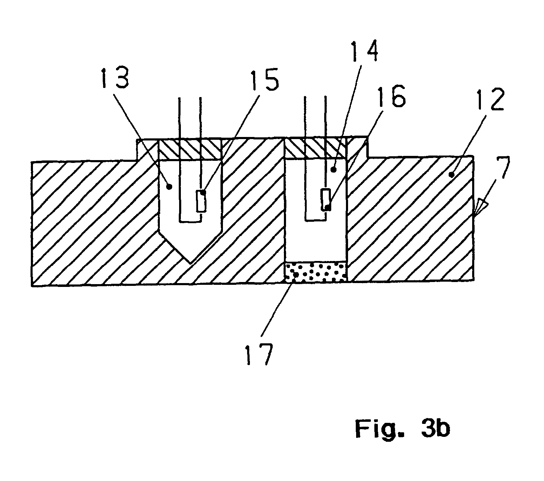

Figur 3a zeigt schematisch den Aufbau eines an sich bekannten Begasungsbrutschrankes, bei dem der Nullpunktabgleich der Wärmeleitfähigkeits-Meßzelle durchgeführt wird. Ein solcher Begasungsbrutschrank ist beispielsweise aus den eingangs genannten DE 29 24 446 C2 oder DE 33 15 085 bekannt, so daß auf nähere Erläuterungen zur Gasströmung und Regelung von Temperatur, Begasung und Feuchtigkeit verzichtet werden kann.

Gemäß Figur 3a wird zu Beginn des Nullpunktabgleichs die schematisch dargestellte Tür 1 des Brutschranks 2 geöffnet. Parallel werden die zwischen Gasversorgung 3 und Gasaustritt 4 angeordneten Gasventile 5 geschlossen, wobei die Tür nach Ablauf einer Mindestöffnungszeit von ca. 30 Sekunden wieder geschlossen wird. Die Gasversorgung 3 enthält beispielsweise eine CO₂-Druckflasche, weitere Flaschen können beispielsweise reinen Sauerstoff oder Stickstoff enthalten. Zwischen den Ventilen 5 und dem Eintritt in den Innenraum 6 des Brutschranks 2 können noch zusätzlich Filter und Vorwärmer eingesetzt sein, die hier jedoch zwecks besserer Klarheit weggelassen sind.

Im oberen Bereich des Innenraums 6 ist schematisch die Wärmeleitfähigkeits-Zelle 7 dargestellt, welche anhand Figur 3b näher erläutert wird.

Weiterhin ist der Innenraum des Brutschranks 2 über Leitung 9 mit einer Verdampfereinheit 8 verbunden, so daß neben einer Temperaturregelung auch eine Regelung der relativen Feuchte zur Erzeugung einer elektronisch geregelten Innenatmosphäre des Brutschranks vorgesehen ist. Da sowohl die Gasversorgung als auch die Verdampfervorrichtung zusammen mit ihren Meß- und Regelungsinstrumenten aus der oben genannten DE 29 24 446 C2 bzw. der DE 33 15 085 C2 ausreichend bekannt sind, wird auf eine nähere Erläuterung hierzu verzichtet.

Gemäß Figur 3b weist die Meßzelle 7 ein Metall-Volumen 12 auf, welches mittels thermischer Regelung auf einem vorgegebenen Temperaturpegel beispielsweise 37° C gehalten wird; Metall-Volumen 12 weist zwei in Abstand zueinander angeordnete Bohrungen 13, 14 auf, die jeweils einen NTC-Widerstand 15, 16 enthalten; die als Referenz-Seite dienende Bohrung 13 ist gegenüber der äußeren Umgebung gasdicht abgeschlossen, während die zur Messung dienende Bohrung 14 mittels eines Filters 17 eine Gasverbindung zum Innenraum 6 des Brutschrankes gemäß Figur 3a aufweist. Von den NTC-Widerständen 15, 16, die auf einem gegenüber dem Metall-Volumen wesentlich höheren Temperaturpegel betrieben werden, geht ein Wärmefluß in Richtung Metallvolumen 12 aus, wobei von NTC-Widerstand 15 ein konstanter Wärmefluß vom Heißleiter zur isothermen Senke des Metallvolumens 12 strömt. Im Bereich der Meßseite der Bohrung 14 wird über Filter 17 der Wärmefluß durch die Atmosphäre beeinflußt, wobei eventuelle Temperaturschwankungen auf der Meßseite mit Hilfe der konstant gehaltenen thermischen Verhältnisse so nachgeregelt werden, daß die Temperaturschwankungen durch die Referenz-Seite kompensiert werden.

Im vorliegenden Beispiel wird zum Vergleich von Referenz-Seite und Meßseite eine übliche Regelung vorgenommen wie sie aus dem eingangs angegebenen Stand der Technik bekannt ist.

## Patentansprüche

1. Verfahren zum Nullpunktabgleich einer Wärmeleitfähigkeits-Messzelle in einem Begasungsbrutschrank mit einer CO₂ enthaltenden Nutzraumatmosphäre, die mit Hilfe der Messzelle unter Verwendung eines Referenzgases geregelt wird, wobei ein Nullpunktabgleich in der feuchten Nutzraumatmosphäre durchgeführt, das Ergebnis gespeichert und für die Nullpunktkorrekturen der folgenden Messungen herangezogen wird, und wobei vor dem Nullpunktabgleich ein vorgegebenes Zeitintervall zur Einstellung eines stabilen Feuchtigkeitsniveaus abgewartet wird,
**dadurch gekennzeichnet,**
**dass** in einer ersten Phase (S1,S2, S3, S4, S5, S6, S7, S8) dem Nutzraum für eine bestimmte Zeit Luft zugeführt und dann der Nutzraum gegenüber der Umgebungsatmosphäre sowie gegen weitere Gaszufuhr abgeschlossen wird,
**dass** anschließend in einer zweiten Phase (S9) ein CO₂-Messwert ermittelt und ein erster Nullpunktabgleich unter Zwischenspeicherung dieses CO₂-Messwertes durchgeführt wird, dass in einer dritten Phase (S10) ein Feuchteaufbau im Inneren des Brutschrankes erfolgt und ein weiterer CO₂-Messwert solange ermittelt wird (S11, S12, S13, S14), bis dieser weitere CO₂-Messwert innerhalb eines vorgegebenen weiteren Toleranzbereichs verbleibt,
**dass** in einer vierten Phase (S15, S16) ein erneuter Nullpunktabgleich mittels des weiteren CO₂-Messwertes erfolgt, und
**dass** anschließend (S17, S18, S19, S20) der Abgleichvorgang abgeschlossen und die CO₂-Gasventile freigegeben werden, sofern ein vorgegebener Abgleichsbereich des ersten und und des weiteren CO₂-Messwertes nicht überschritten worden ist.

2. Verfahren nach Anspruch 1, **dadurch gekennzeichnet, dass** nach Aufruf des Start-Schritts (S1) alle CO₂-Messwerte der Messzelle intern auf Null zurückgesetzt werden.

3. Verfahren nach Anspruch 1 oder 2, **dadurch gekennzeichnet, dass** nach Ermittlung des ersten CO2-Messwertes die Atmosphäre des Proben-Innenraums befeuchtet wird und nach Erreichen eines Beharrungszustands der Feuchte der anschließend ermittelte weitere CO₂-Messwert über eine vorgegebene Zeit auf die Einhaltung des Toleranzbereichs von ± 0,2 Vol-% CO2 überprüft wird.

4. Verfahren nach Anspruch 3, **dadurch gekennzeichnet, dass** bei Über -oder Unterschreiten eines vorgegebenen CO2-Meßwertes der Nullpunktabgleich als fehlerhaft abgebrochen wird.

5. Verfahren nach Anspruch 4, **dadurch gekennzeichnet, dass** ein neuer Startvorgang zum Nullpunktabgleich durchgeführt wird.

6. Verfahren nach einem der Ansprüche 1 bis 5, **dadurch gekennzeichnet, dass** als Referenzgas ein Luft-Volumen eingesetzt wird.

7. Verfahren nach Anspruch 6, **dadurch gekennzeichnet, dass** Referenzgas aus einem strömenden Luft-Volumen eingesetzt wird.

## Claims

1. A method of zero-point adjustment of a thermal conductivity measuring cell in an incubator with a CO₂ containing chamber atmosphere, which is controlled by means of the measuring cell using a reference gas, whereby a zero-point adjustment is carried out in the humid chamber atmosphere, the result is stored and used for the zero-point corrections of the subsequent measurements, and whereby, prior to the zero-point adjustment, a predetermined timing interval is awaited for equilibration of a stable humidity level,
**characterized in that**
in a first phase (S1, 52, 53, 54, S5, 56, 57, 58), air is supplied to the chamber for a predetermined time and then the chamber is sealed off with respect to ambient atmosphere and further gas supply, subsequently in a second phase (S9), a CO₂ value is established and a first zero-point adjustment is carried out with intermediate storage of this CO₂ value,
in a third phase (S10), a humidity build-up takes place in the interior of the incubator and a second CO₂ value is established (S11, S12, S13, S14) until this second CO₂ value remains within a second predetermined tolerance band,
in a fourth phase (S15, S16), a zero-point adjustment takes place again by means of the second CO₂ value, and
subsequently (S17, S18, S19, S20) the adjustment procedure is completed and the CO₂ gas valves are opened, if a predetermined adjustment band of the first and the second CO₂ value has not been exceeded.

2. The method according to claim 1,
**characterized in that**
after call of the start step (51) all CO₂ values of the measuring cell are internally reset to zero.

3. The method according to claim 1 or 2,
**characterized in that**
after establishing the first CO₂ value the atmosphere of the sample interior is humidified and after reaching a steady-state of humidity the subsequently established second CO₂ value is tested for a predetermined time for compliance with the tolerance band of ± 0.2 vol-% CO₂.

4. The method according to claim 3,
**characterized in that**
the zero-point adjustment is terminated as faulty, if a predetermined CO₂ value is exceeded or undershot.

5. The method according to claim 4,
**characterized in that**
a new start procedure for the zero-point adjustment is carried out.

6. The method according to one of claims 1 to 5,
**characterized in that**
an air volume is used as a reference gas.

7. The method according to claim 6,
**characterized in that**
a reference gas is used from a streaming air volume.

## Revendications

1. Procédé de compensation du zéro d'une cellule de mesure de conductibilité thermique dans une armoire d'incubation à gaz avec une atmosphère utile contenant du CO₂, qui est régulée à l'aide de la cellule de mesure en utilisant un gaz de référence, dans lequel une compensation du zéro est conduite dans l'atmosphère utile humide, le résultat enregistré et utilisé pour les corrections du zéro des mesures suivantes, et dans lequel on attend un intervalle de temps prédéterminé avant la compensation du zéro afin d'instaurer un niveau d'humidité stable,
***caractérisé en ce que*,** dans une première phase (S1, S2, S3, S4, S5, S6, S7, S8), de l'air est introduit dans la chambre utile pendant un temps déterminé, puis la chambre utile est fermée par rapport à l'atmosphère environnante et par rapport à une autre introduction de gaz,
***en ce qu***'ensuite, dans une deuxième phase (S9), une valeur de mesure de CO₂ est déterminée et une première compensation du zéro est effectuée en enregistrant cette valeur de mesure de C02,
***en ce que*,** dans une troisième phase (S10), une humidité est établie à l'intérieur de l'armoire d'incubation et une autre valeur de mesure de CO₂ est déterminée (S11, S12, S13, S14), jusqu'à ce que cette nouvelle valeur de mesure de CO₂ demeure dans une autre plage de tolérance déterminée,
***en ce que*,** dans une quatrième phase (S15, S16), une nouvelle compensation du zéro est effectuée au moyen de l'autre valeur de mesure de CO₂, et
***en ce qu***'ensuite (S17, S18, S19, S20), la procédure de compensation est terminée et les vannes de gaz CO₂ sont libérées si une plage de compensation prédéterminée de la première et de l'autre valeur de mesure de CO₂ n'a pas été dépassée.

2. Procédé selon la revendication 1, ***caractérisé en ce qu***'après le lancement de l'étape de départ (S1), toutes les valeurs de mesure de CO₂ de la cellule de mesure sont remises à zéro en interne.

3. Procédé selon la revendication 1 ou 2, ***caractérisé en ce qu***'après la détermination de la première valeur de mesure de CO₂, l'atmosphère de la chambre intérieure à échantillons est humidifiée et, une fois atteint un état d'équilibre de l'humidité, l'autre valeur de mesure de CO₂ déterminée ensuite est vérifiée pendant une période prédéterminée pour s'assurer du respect de la plage de tolérance de ± 0,2 % vol. de CO₂.

4. Procédé selon la revendication 3, ***caractérisé en ce qu***'en cas de dépassement vers le haut ou vers le bas d'une valeur de mesure de CO₂ prédéterminée, la compensation de zéro est jugée erronée et interrompue.

5. Procédé selon la revendication 4, ***caractérisé en ce qu***'une nouvelle opération de départ en vue d'une compensation du zéro est effectuée.

6. Procédé selon l'une quelconque des revendications 1 à 5, ***caractérisé en ce que*** l'on utilise un volume d'air comme gaz de référence.

7. Procédé selon la revendication 6, ***caractérisé en ce que*** l'on utilise un gaz de référence provenant d'un volume d'air en écoulement.
